# EUROPÄISCHE PATENTANMELDUNG

(11) **EP 1 913 934 A1**
(43) Veröffentlichungstag der Anmeldung: **23.04.2008**
(21) Anmeldenummer: 07017483.4
(22) Anmeldetag: 06.09.2007
(51) Int. Cl.: A61K 9/00, A61K 36/185, A61K 36/537, A61K 36/31, A61K 36/55, A61K 36/535, A61K 36/57, A61K 47/32, A61K 47/10

(54) **Emulgatorfreies opthalmisches Präparat enthaltend Plflanzenöl**

(30) Priorität: 18.10.2006 DE 102006049091
(71) Anmelder: Dr. GERHARD MANN chem.-pharm. Fabrik GmbH, D-13581 Berlin (DE)
(72) Erfinder: Keßler, Christoph, 10623 Berlin (DE); Bellmann, Günther, 13593 Berlin (DE); Claus-Herz, Gudrun, 14167 Berlin (DE)
(74) Vertreter: Maiwald Patentanwalts GmbH

(57) **Zusammenfassung**

Die Erfindung betrifft ein steriles tropfbares mehrphasiges emulgatorfreies ophthalmisches Präparat, insbesondere Gelpräparat, enthaltend wenigstens eine flüssige wässrige Phase und wenigstens eine flüssige hydrophobe Phase, wobei das Präparat wenigstens ein Pflanzenöl ausgewählt aus der Gruppe umfassend Kiwisamenöl, Leinöl, Walnussöl, Rapsöl, Chiaöl, Perillaöl und/oder Hanföl umfasst.

## Beschreibung

Die vorliegende Erfindung betrifft ein emulgatorfreies ophthalmisches Präparat. Das ophthalmische Präparat kann beispielsweise für die Behandlung der Symptomatik des trockenen Auges ("dry eye syndrome") verwendet werden.

Trockene Augen treten auf, wenn das empfindliche System von Tränenproduktion und Tränenverteilung gestört ist. Es ist allgemein bekannt, dass die Augen beispielsweise durch intensive Bildschirmarbeit, das Tragen von Kontaktlinsen oder durch trockene Raumluft beispielsweise durch Klimaanlagen häufig brennen, jucken oder tränen. Hierfür ist oft eine Funktionsstörung des Tränenfilms die Ursache, ausgelöst durch zu hohe Verdunstung oder zu geringe Tränenproduktion. Aber auch durch hormonelle Umstellungen im Alter, durch Einnahme von Medikamenten oder durch innere Erkrankungen wie dem Sjögren-Syndrom, Rheuma oder Diabetes können Benetzungsstörungen des Auges begünstigt werden.

Häufig ist dieses ein chronisches Phänomen, das einer fortlaufenden Behandlung bedarf. Die Funktionsstörungen des Tränenfilms können sich in einer Vielzahl von Symptomen äußern. Häufige Beschwerden von Patienten, die an trockenem Auge leiden, sind Trockenheitsgefühl, Fremdkörpergefühl, Sandkorngefühl oder Druck auf den Augenlidern.

Es ist im Stand der Technik bekannt, bei gereizten oder entzündeten Augen, insbesondere bei "trockenem Auge", Keratokonjunktivitis sicca, Präparate zur Behandlung der Beschwerden einzusetzen.

Beispielsweise offenbart die EP 0 817 610 A1 ein steriles tropfbares mehrphasiges emulgatorfreies ophthalmisches Präparat, das wenigstens eine flüssige wässrige Phase und wenigstens eine flüssige hydrophobe Phase enthält. Jedoch zeigt das in der EP 0 817 610 A1 offenbarte Präparat keine optimalen Ergebnisse bei der Unterstützung der Tränensekretion des Auges.

Es ist die Aufgabe der vorliegenden Erfindung eine Zusammensetzung zur Verfügung zu stellen, die wenigstens einen der vorgenannten Nachteile des Standes der Technik überwindet. Insbesondere ist es Aufgabe der vorliegenden Erfindung, eine Zusammensetzung zur Verfügung zu stellen, die zur Anwendung bei trockenem Auge geeignet ist.

Die Aufgabe wird gelöst durch ein steriles tropfbares mehrphasiges emulgatorfreies ophthalmisches Präparat, insbesondere Gelpräparat, enthaltend wenigstens eine flüssige wässrige Phase und wenigstens eine flüssige hydrophobe Phase, wobei das Präparat wenigstens ein Pflanzenöl ausgewählt aus der Gruppe umfassend Kiwisamenöl, Leinöl, Walnussöl, Rapsöl, Chiaöl, Perillaöl und/oder Hanföl umfasst.

Vorteilhafte Ausgestaltungen des erfindungsgemäßen ophthalmischen Präparats sind in den Unteransprüchen angegeben.

"Emulgatorfrei" bedeutet im Sinne dieser Erfindung, dass das ophthalmische Präparat ausschließlich Inhaltsstoffe aufweist, die keine Emulgatoren im Sinne der vorliegenden Erfindung sind. Insbesondere kann das Präparat gemäß der vorliegenden Erfindung Stoffe ausgewählt aus der Gruppe umfassend ein Pflanzenöl ausgewählt aus der Gruppe umfassend Kiwisamenöl, Leinöl, Walnussöl, Rapsöl, Chiaöl, Perillaöl und/oder Hanföl, Polyacrylsäure, polymere Acrylsäurederivate, vorzugsweise Carbomer, wasserlösliche Phosphatsalze, vorzugsweise Na₂HPO₄ x 12 H₂O, eine Vitamin A-Komponente, vorzugsweise Vitamin A-palmitat, Vitamin E, vorzugsweise gemischte Tocopherole, Sorbitol, Natriumhydroxid, Konservierungsmittel, vorzugsweise Cetrimid und/oder Wasser aufweisen, wobei es sich bei diesen Stoffen im Sinne der vorliegenden Erfindung nicht um Emulgatoren handelt.

Die Stoffe, die das Präparat gemäß der vorliegenden Erfindung enthalten kann, insbesondere ein Pflanzenöl ausgewählt aus der Gruppe umfassend Kiwisamenöl, Leinöl, Walnussöl, Rapsöl, Chiaöl, Perillaöl und/oder Hanföl, Polyacrylsäure, polymere Acrylsäurederivate, vorzugsweise Carbomere, wasserlösliche Phosphatsalze, vorzugsweise Na₂HPO₄ x 12 H₂O eine Vitamin A-Komponente, vorzugsweise Vitamin A-palmitat, Vitamin E, vorzugsweise gemischte Tocopherole, Sorbitol, Natriumhydroxid, Konservierungsmittel, vorzugsweise Cetrimid und/oder Wasser, werden in Mengen eingesetzt, dass die vorgenannten Stoffe keine emulgierende Wirkung aufweisen. In bevorzugten Ausführungsformen enthält das ophthalmische Präparat darüber hinaus keine weiteren Emulgatoren oder in Mengen, dass die vorgenannten Stoffe keine emulgierende Wirkung aufweisen.

Es hat sich überraschend gezeigt, dass das erfindungsgemäße Präparat die Symptomatik des trockenen Auges positiv beeinflussen kann. Ein besonderer Vorteil des erfindungsgemäßen Präparats liegt darin, dass insbesondere die eigene Tränenproduktion des Auges unterstützt werden kann.

Ein Vorteil der Pflanzenöl ausgewählt aus der Gruppe umfassend Kiwisamenöl, Leinöl, Walnussöl, Rapsöl, Chiaöl, Perillaöl und/oder Hanföl ist, dass diese einen geringen oder keinen Eigengeruch aufweisen. Dies stellt insbesondere einen Vorteil gegenüber der Verwendung von Fischöl oder Algenöl dar, die einen intensiven Eigengeruch aufweisen und bei einer topischen Anwendung einen für den Patienten unangenehmen Geruch verursachen und daher wenig geeignet sind.

Bevorzugt verwendbar sind Pflanzenöle ausgewählt aus der Gruppe umfassend Kiwisamenöl, Leinöl, Rapsöl und/oder Hanföl, vorzugsweise verwendbar sind Pflanzenöle ausgewählt aus der Gruppe umfassend Kiwisamenöl und/oder Rapsöl, besonders bevorzugt ist Kiwisamenöl. Leinöl wird auch als Leinsamenöl oder Flachsöl bezeichnet.

Die Pflanzenöle können vorteilhafter Weise die Verdunstung der wässrigen Komponente verhindern und die Verweildauer des Tränenfilms oder des Präparats im Auge verbessern.

Bevorzugt verwendbar sind natürliche Pflanzenöle, insbesondere native Öle, vorzugsweise nicht raffinierte Öle, beispielsweise verwendbar sind kalt gepresste Öle. Ein Vorteil der Verwendung von natürlichen Pflanzenölen liegt in der ausgezeichneten Verträglichkeit. Ein weiterer Vorteil natürlicher Pflanzenöle liegt in der natürlichen Zusammensetzung der Inhaltsstoffe, insbesondere der enthaltenen Fettsäuren. Ein besonderer Vorteil ist, dass die Bioverfügbarkeit der Inhaltsstoffe, beispielsweise der Fettsäuren, natürlicher Pflanzenöle höher sein kann. Ein weiterer Vorteil natürlicher Pflanzenöle liegt darin, dass natürliche Pflanzenöle die Inhaltsstoffe, beispielsweise Fettsäuren, in einer ausgewogenen Zusammensetzung aufweisen. Insbesondere verursachen natürliche Öle vorzugsweise keine ungünstigen oder überschiessenden Umwandlungsreaktionen der enthaltenen Fettsäuren in andere Fettsäuren wie es beispielsweise zu hohe oder unausgewogene Gaben einzelner Fettsäuren, insbesondere synthetischer Zusammensetzung, bewirken können.

Das ophthalmische Präparat enthält wenigstens ein Pflanzenöl ausgewählt aus der Gruppe umfassend Kiwisamenöl, Leinöl, Walnussöl, Rapsöl, Chiaöl, Perillaöl und/oder Hanföl. Das ophthalmische Präparat kann auch Gemische verwendbarer Pflanzenöle aufweisen.

In bevorzugten Ausführungsformen enthält das ophthalmische Präparat wenigstens ein Pflanzenöl ausgewählt aus der Gruppe umfassend Kiwisamenöl, Leinöl, Walnussöl, Rapsöl, Chiaöl, Perillaöl und/oder Hanföl im Bereich von ≥ 0,01 Gew.-% bis ≤ 10 Gew.-%, bezogen auf das Gesamtgewicht des Präparats, bevorzugt im Bereich von ≥ 0,1 Gew.-% bis ≤ 5 Gew.-%, vorzugsweise im Bereich von ≥ 0,5 Gew.-% bis ≤ 2 Gew.-%, besonders bevorzugt etwa 1 Gew.-% Pflanzenöl.

Besonders bevorzugt verwendbar sind Pflanzenöl-Extrakte, beispielsweise CO₂-Extrakte Vorzugsweise enthalten verwendbare Pflanzenöl-Extrakte keine technischen Zusatzstoffe. Weiter bevorzugt sind verwendbare Pflanzenöl-Extrakte nicht formuliert. Unter dem Begriff "Extrakt" werden im Sinne dieser Erfindung Auszüge von flüssiger und/oder öliger Beschaffenheit von Pflanzen oder deren Teilen verstanden. Extrakte können beispielsweise unter Verwendung von Lösemittel ausgewählt aus der Gruppe umfassend Wasser, Kohlendioxid und/oder Kohlensäure gewonnen werden.

Verwendbare Pflanzenöle oder Pflanzenöl-Extrakte können Mittel zur Stabilisierung enthalten. Beispielsweise können verwendbare Pflanzenöl-Extrakte zur Stabilisierung vorzugsweise natürliche Antioxidantien enthalten, bevorzugt ausgewählt aus der Gruppe umfassend Vitamin E, vorzugsweise Tocopherole und/oder Diterpenphenole. Vorzugsweise können verwendbare Pflanzenöl-Extrakte zur Stabilisierung Extrakte aus Rosmarin enthalten, die Diterpenphenole, Lecithin und/oder Tocopherole, vorzugsweise gemischte Tocopherole, enthalten können.

Bei Rosmarin-Extrakt, Diterpenphenol, Lecithin und/oder Tocopherol handelt es sich im Sinne der vorliegenden Erfindung nicht um Emulgatoren, oder Rosmarin-Extrakt, Diterpenphenole, Lecithin und/oder Tocopherole sind in Mengen enthalten, dass die Stoffe keine emulgierende Wirkung aufweisen.

Verwendbares Pflanzenöl enthält vorzugsweise wenigstens eine einfach oder mehrfach ungesättigte Fettsäure, vorzugsweise wenigstens eine mehrfach ungesättigte Fettsäure.

Verwendbares Pflanzenöl enthält bevorzugt wenigstens eine Omega-3-Fettsäure. Bevorzugte Omega-3-Fettsäuren sind ausgewählt aus der Gruppe umfassend α-Linolensäure und/oder Stearidonsäure. Eine besonders bevorzugte Omega-3-Fettsäuren ist α-Linolensäure. Beispielsweise enthalten verwendbare Pflanzenöle ausgewählt aus der Gruppe umfassend Kiwisamenöl, Leinöl und/oder Rapsöl α-Linolensäure.

Weiterhin enthält verwendbares Pflanzenöl vorzugsweise wenigstens eine Omega-6-Fettsäure. Bevorzugte Omega-6-Fettsäuren sind ausgewählt aus der Gruppe umfassend Linolsäure, γ-Linolensäure und/oder Dihomo-γ-Linolensäure. Eine besonders bevorzugte Omega-6-Fettsäure ist Linolsäure.

Ohne auf eine bestimmte Theorie festgelegt zu sein, wird vermutet, dass die in Pflanzenölen enthaltenen Omega-3-Fettsäuren und/oder Omega-6-Fettsäuren einen maßgeblichen Einfluss auf die verbesserte Wirkung bei der Symptomatik des Trockenen Auges haben.

Verwendbares Pflanzenöl kann neben der vorzugsweise enthaltenen wenigstens einen mehrfach ungesättigten Fettsäure, vorzugsweise Omega-3-Fettsäure und/oder Omega-6-Fettsäure, weitere Fettsäuren enthalten. Insbesondere können natürliche Pflanzenöle Gemische verschiedener gesättigter und/oder ungesättigter Fettsäuren enthalten. Beispielsweise kann ein verwendbares Pflanzenöl weiterhin eine oder mehrere gesättigte oder ungesättigte Fettsäuren, vorzugsweise C₁₆- und/oder C₁₈-Fettsäuren, bevorzugt ausgewählt aus der Gruppe umfassend Palmitinsäure, Palmitoleinsäure, Stearinsäure, Ölsäure und/oder Vaccensäure enthalten.

Ohne auf eine bestimmte Theorie festgelegt zu sein, wird vermutet, dass die in natürlichen Pflanzenölen enthaltenen Gemische verschiedener gesättigter und/oder ungesättigter Fettsäuren einen bedeutenden Einfluss auf die verbesserte Wirkung bei der Symptomatik des Trockenen Auges haben.

In bevorzugten Ausführungsformen enthält das ophthalmische Präparat wenigstens eine Omega-3-Fettsäure vorzugsweise α-Linolensäure und wenigstens eine Omega-6-Fettsäure vorzugsweise Linolsäure. In besonders bevorzugten Ausführungsformen enthält das ophthalmische Präparat wenigstens eine Omega-3-Fettsäure, vorzugsweise α-Linolensäure, und wenigstens eine Omega-6-Fettsäure, vorzugsweise Linolsäure, im Bereich von ≥ 0,01 Gew.-% bis ≤ 8 Gew.-%, bezogen auf das Gesamtgewicht des Präparats, bevorzugt im Bereich von ≥ 0,1 Gew.-% bis ≤ 4 Gew.-%, vorzugsweise im Bereich von ≥ 0,2 Gew.-% bis ≤ 1 Gew.-%, besonders bevorzugt im Bereich von ≥ 0,5 Gew.-% bis ≤ 0,8 Gew.-%.

In bevorzugten Ausführungsformen ist das Pflanzenöl ausgewählt aus der Gruppe umfassend Kiwisamenöl, Leinöl, Walnussöl, Rapsöl, Chiaöl, Perillaöl und/oder Hanföl ein ophthalmisch und/oder ophthalmologisch akzeptables, organisches Öl. Das ophthalmisch akzeptable organische Pflanzenöl bildet die flüssige hydrophobe Phase des erfindungsgemäßen Präparats aus.

Von Vorteil ist, dass das ophthalmische Präparat in bevorzugten Ausführungsformen bei der Anwendung im Auge der dreiphasigen Zusammensetzung des natürlichen Tränenfilms im Auge, Mucinschicht, wässrige Phase und Lipidschicht, nahe kommt und den natürlichen Tränenfilm substituieren kann. Insbesondere wird dies in vorteilhafter Weise unabhängig davon ermöglicht, welche Phase des natürlichen Tränenfilms in zu geringem Maße vorhanden ist oder fehlt.

Ein weiterer überraschender Effekt des erfindungsgemäßen ophthalmischen Präparats ist, dass das Präparat die eigene Tränenproduktion des Auges unterstützen kann. Ein besonderer Vorteil des erfindungsgemäßen ophthalmischen Präparats kann in bevorzugten Ausführungsformen dadurch zur Verfügung gestellt werden, dass das ophthalmische Präparat nicht darauf beschränkt ist den Tränenfilm des Auges zu substituieren sondern ebenfalls die eigene Tränenproduktion und -sekretion des Auges positiv beeinflussen kann.

Vorzugsweise enthält das mehrphasige Präparat die wässrige Phase als kontinuierliche Phase, und die flüssige hydrophobe Phase, vorzugsweise eine flüssige Ölphase, als darin dispergierte Tröpfchen, vorzugsweise in Form feinstverteilter Tröpfchen. Das ophthalmische Präparat ist vorzugsweise wenigstens zweiphasig ausgebildet. Das ophthalmische Präparat umfasst vorzugsweise weiterhin eine polymere gelbildende Komponente in der wässrigen Phase. Entsprechend enthält das erfindungsgemäße Präparat vorzugsweise eine zweiphasige, eine flüssige wässrige Phase und eine flüssige hydrophobe Phase umfassende, Trägerflüssigkeit oder Gelbasis.

Die wässrige Phase des ophthalmischen Präparats wird bevorzugt dadurch ausgebildet, dass das ophthalmische Präparat einen bedeutenden Wasseranteil aufweist. Vorzugsweise umfasst das Präparat einen Wasseranteil im Bereich von ≥ 60 Gew.-% bis ≤ 98 Gew.-%, bezogen auf das Gesamtgewicht des Präparats, bevorzugt im Bereich von ≥ 90 Gew.-% bis ≤ 96 Gew.-%, besonders bevorzugt enthält das Präparat etwa ≥ 94 Gew.-% bis ≤ 95 Gew.-% Wasser. Bevorzugt verwendbar ist Wasser für Injektionszwecke.

Die wässrige Phase des Präparats kann in bevorzugten Ausführungsformen die wässrige Phase des natürlichen Tränenfilms, die die Hornhaut befeuchtet, nachbilden und eine reinigende und schützende Funktion ausüben.

Vorteilhafter Weise ist das erfindungsgemäße ophthalmische Präparat besonders gut verträglich. Weiterhin von Vorteil ist, dass das erfindungsgemäße ophthalmische Präparat zu keiner oder einer nur sehr geringen Reizung oder Unannehmlichkeit bei der Anwendung am Auge führt. Dies bedeutet einen großen Vorteil, da eine Applikation von Präparaten am Auge vielfach zu einem sehr unangenehmen Gefühl des "Brennens" führt.

In bevorzugten Ausführungsformen enthält das ophthalmische Präparat wenigstens eine polymere gelbildende Komponente, vorzugsweise eine Polyacrylsäure und/oder ein polymeres Acrylsäurederivat, bevorzugt ein Carbomer.

Bevorzugte Polyacrylsäuren weisen beispielsweise ein Molekulargewicht im Bereich von etwa 3 bis 5 Millionen Dalton auf. Besonders bevorzugt verwendbare Polyacrylsäuren oder polymere Acrylsäurederivate sind die unter der INCI-Bezeichnung Carbomer bezeichneten quervernetzten Acrylsäurepolymere, vorzugsweise hydrophobisch modifizierte quervernetzte Acrylsäurepolymere. Bevorzugte Carbomere sind unter dem Handelsnamen Carbopol® beispielsweise bei der Firma Noveon Inc. erhältlich. Besonders bevorzugte Carbomere sind Acrylsäure-Homopolymere, beispielsweise Carbopol®-Homopolymere, besonders bevorzugt verwendbar ist Carbopol® 980 NF, weiterhin bevorzugt verwendbar ist Carbopol® 940 NF. Die Begriffe Polyacrylsäure und polymere Acrylsäure werden im Sinne dieser Anmeldung synonym verwendet.

Vorzugsweise enthält das ophthalmische Präparat die polymere gelbildende Komponente, vorzugsweise Polyacrylsäure und/oder ein polymeres Acrylsäurederivat, insbesondere bevorzugt ein Carbomer, im Bereich von ≥ 0,1 Gew.-% bis ≤ 3 Gew.-%, bezogen auf das Gesamtgewicht des Präparats, bevorzugt im Bereich von ≥ 0,1 Gew.-% bis ≤ 1 Gew.-%, besonders bevorzugt im Bereich von ≥ 0,15 Gew.-% bis ≤ 0,5 Gew.-%, ganz besonders bevorzugt etwa 0,2 Gew.-%.

Bevorzugte Ausführungsformen des erfindungsgemäßen Präparats, insbesondere auf Gelbasis, mit Gehalten an Gelbildner in der wässrigen Phase im Bereich von ≥ 0,1 Gew.-% bis ≤ 3 Gew.-%, bezogen auf das Gesamtgewicht des Präparats, insbesondere etwa 0,2 Gew.-%, enthalten wenigstens ein Pflanzenöl ausgewählt aus der Gruppe umfassend Kiwisamenöl, Leinöl, Walnussöl, Rapsöl, Chiaöl, Perillaöl und/oder Hanföl im Bereich von ≥ 0,5 Gew.-% bis ≤ 10 Gew.-%, insbesondere etwa 1 Gew.-% Pflanzenöl ausgewählt aus der Gruppe umfassend Kiwisamenöl, Leinöl, Walnussöl, Rapsöl, Chiaöl, Perillaöl und/oder Hanföl.

Ein weiterer besonderer Vorteil des Präparats wird dadurch verwirklicht, dass das ophthalmische Präparat Pflanzenöl enthalten kann, ohne dass gleichzeitig Emulgatoren eingesetzt werden müssen. Das hat den großen Vorteil, dass eine Reizung des Auges durch Emulgatoren vermieden werden kann.

Vorzugsweise liegt das Pflanzenöl ausgewählt aus der Gruppe umfassend Kiwisamenöl, Leinöl, Walnussöl, Rapsöl, Chiaöl, Perillaöl und/oder Hanföl in dem erfindungsgemäßen Präparat in Form von feinstverteilten Tröpfchen vor. Ohne auf eine bestimmte Theorie festgelegt zu sein, wird angenommen, dass das Vorliegen des Pflanzenöls in dem erfindungsgemäßen Präparat in Form von feinstverteilten Tröpfchen eine überraschende Stabilität auch über lange Lagerzeiten ohne Emulgatorzusatz ermöglicht.

In besonders bevorzugten Ausführungsformen enthält das ophthalmische Präparat ein Phosphatsalz, bevorzugt ein wasserlösliches Phosphatsalz. Vorzugsweise ist das Phosphatsalz ausgewählt aus der Gruppe umfassend Alkaliphosphatsalze, Erdalkaliphosphatsalze, Ammoniumphosphatsalze, deren Mono- oder Dihydrogenphosphate und/oder Mischungen davon. Bevorzugt enthält das ophthalmische Präparat ein Phosphatsalz ausgewählt aus der Gruppe umfassend Natriumdihydrogenphosphat, Natriummonohydrogenphosphat, Kaliumdihydrogenphosphat, Kaliummonohydrogenphosphat und/oder Mischungen davon, besonders bevorzugt Na₂HPO₄ x 12 H₂O.

Vorteilhafter Weise ermöglicht die Verwendung von Phosphatsalzen niedrige Bereiche der Viskosität einzustellen. Insbesondere ist von Vorteil, dass ein Präparat mit geringer Viskosität die Applizierbarkeit für den Patienten deutlich verbessert.

Vorzugsweise enthält das ophthalmische Präparat Na₂HPO₄ x 12 H₂O im Bereich von ≥ 0,01 Gew.-% bis ≤ 5,0 Gew.-%, bezogen auf das Gesamtgewicht des Präparats, bevorzugt im Bereich von ≥ 0,05 Gew.-% bis ≤ 0,5 Gew.-%, vorzugsweise im Bereich von ≥ 0,08 Gew.-% bis ≤ 0,2 Gew.-%, besonders bevorzugt etwa 0,1 Gew.-% Na₂HPO₄ x 12 H₂O.

In bevorzugten Ausführungsformen enthält das erfindungsgemäße Präparat keine Arzneistoffe oder pharmazeutisch aktiven Substanzen wie antivirale Mittel, steroidale und nicht-steroidale entzündungshemmende Verbindungen oder Corticosteroide, Antibiotika, Antimykotika, Narkotika, entzündungshemmender Agenzien oder antiallergische Agenzien. Pharmazeutisch aktive Substanzen im Sinne der Erfindung sind insbesondere nicht Vitamin A und Vitamin E.

In bevorzugten Ausführungsformen kann das ophthalmische Präparat weiterhin wenigstens einen ophthalmischen Wirkstoff enthalten, vorzugsweise ausgewählt aus der Gruppe umfassend Vitamin A und/oder Vitamin E, bevorzugt gemischte Tocopherole. Bevorzugt enthält das ophthalmische Präparat Vitamin A-palmitat und/oder Tocopherole, bevorzugt gemischte Tocopherole. Über die Verwendung von Vitaminen wie Vitamin A und/oder Vitamin E, bevorzugt gemischte Tocopherole, hinaus sind vorzugsweise keine pharmazeutisch aktiven Komponenten in dem erfindungsgemäßen Präparat vorgesehen.

Das ophthalmische Präparat kann Vitamin E und/oder Tocopherole als Antioxidanz enthalten. Vorzugsweise ist vorgesehen, dass der Gehalt des Präparats an Vitamin E und/oder Tocopherole 0,1 Gew.-%, vorzugsweise 0,01 Gew.-%, bezogen auf das Gesamtgewicht des Präparats, nicht überschreitet. Bevorzugt liegt der Gehalt an des Präparats an Vitamin E und/oder Tocopherol im Bereich von ≥ 0,001 Gew.-% bis ≤ 0,1 Gew.-%, bezogen auf das Gesamtgewicht des Präparats, vorzugsweise im Bereich von ≥ 0,003 Gew.-% bis ≤ 0,03 Gew.-%, noch bevorzugt im Bereich von ≥ 0,004 Gew.-% bis ≤ 0,02 Gew.-%, besonders bevorzugt im Bereich von ≥ 0,005 Gew.-% bis ≤ 0,01 Gew.-%.

In bevorzugten Ausführungsformen umfasst das ophthalmische Präparat ein oder mehrere Mittel zur Einstellung der Isotonizität, vorzugsweise mehrwertige Alkohole bevorzugt ausgewählt aus der Gruppe umfassend Dextrose, Glycerin, Propylenglycol, Sorbitol und/oder Mannitol. Bevorzugt enthält das ophthalmische Präparat Sorbitol. Vorzugsweise weist das Präparat das oder die Mittel zur Einstellung der Isotonizität in einer Menge auf, die ausreicht, um die Zusammensetzung isotonisch mit natürlicher Tränenflüssigkeit bereit zu stellen. In bevorzugten Ausführungsformen umfasst das Präparat Isotonierungsmittel, vorzugsweise Sorbitol, im Bereich von ≥ 0,5 Gew.-% bis ≤ 10 Gew.-%, bezogen auf das Gesamtgewicht des Präparats, bevorzugt im Bereich von ≥ 1 Gew.-% bis ≤ 6 Gew.-%, besonders bevorzugt im Bereich von ≥ 4 Gew.-% bis ≤ 5 Gew.-%.

Vorzugsweise wird der pH-Wert des ophthalmischen Präparats mit Basen, bevorzugt mit Natronlauge eingestellt, wobei die Verwendung einer 2 %igen bis 3 %igen Lösung von Natriumhydroxid in Wasser besonders vorteilhaft ist.

Das ophthalmische Präparat enthält bevorzugt Natriumhydroxid, vorzugsweise im Bereich von ≥ 0,02 Gew.-% bis ≤ 1 Gew.-%, bezogen auf das Gesamtgewicht des Präparats, weiter bevorzugt im Bereich von ≥ 0,05 Gew.-% bis ≤ 0,1 Gew.-%, besonders bevorzugt im Bereich von ≥ 0,06 Gew.-% bis ≤ 0,08 Gew.-%. Die Angabe bezieht sich, wenn nicht anders angegeben, auf den Feststoff Natriumhydroxid.

Das erfindungsgemäße Präparat kann Konservierungsmittel enthalten, vorzugsweise ausgewählt aus der Gruppe umfassend Cetrimid, Benzododeciniumchlorid, Benzalkoniumchlorid, Thiomersal und/oder Alexidin. Bevorzugt enthält das Präparat Konservierungsmittel, vorzugsweise ausgewählt aus der Gruppe umfassend Cetrimid, Benzododeciniumchlorid, Benzalkoniumchlorid und/oder Thiomersal, im Bereich von ≥ 0,001 Gew.-% bis ≤ 0,05 Gew.-%, bezogen auf das Gesamtgewicht des Präparats, besonders bevorzugt im Bereich von ≥ 0,005 Gew.-% bis ≤ 0,01 Gew.-%.

"Cetrimid" ist die übliche Bezeichnung für N-Cetyl-N,N,N-trimethyl-ammoniumbromid, "Benzododeciniumchlorid" für N-Benzyl-N-dodecyl-N,N-dimethyl-ammoniumchlorid, "Benzalkoniumchlorid" für Benzyllauryldimethylammoniumchlorid, "Thiomersal" für das Natriumsalz von 2-(Ethylmercurithio)-benzoesäure.

In bevorzugten Ausführungsformen umfasst das ophthalmische Präparat Cetrimid im Bereich von ≥ 0,005 Gew.-% bis ≤ 0,01 Gew.-%, bezogen auf das Gesamtgewicht des Präparats. In besonders bevorzugten Ausführungsformen ist das Präparat frei von Konservierungsmitteln.

Das erfindungsgemäße Präparat kann als Konservierungsmittel Alexidin enthalten, bevorzugt Alexidinhydrochlorid, vorzugsweise Alexidin x 2HCl. Bevorzugt kann das ophthalmische Präparat Alexidinhydrochlorid im Bereich von ≥ 0,0003 Gew.-% bis ≤ 0,0005 Gew.-% Alexidin HCl, bezogen auf das Gesamtgewicht des Präparats, enthalten. Bei Alexidin handelt es sich im Sinne der vorliegenden Erfindung nicht um einen Emulgator, oder Alexidin ist in Mengen enthalten, dass der Stoff keine emulgierende Wirkung aufweist.

In vorteilhaften Ausführungsformen liegt die Viskosität des Präparats im Bereich von ≥ 300 mPa·s bis ≤ 8000 mPa·s. Vorteilhafter Weise kann das erfindungsgemäße Präparat in einem Viskositätsbereich von ≥ 300 mPa·s bis ≤ 8000 mPa·s, insbesondere in einem Bereich < 2000 mPa·s stabil formuliert werden. Dies ist insbesondere bei Emulsion niedriger Viskosität enthaltend eine hydrophile, beispielsweise wässrige, Phase und eine hydrophobe Phase von Vorteil, da diese üblicherweise rasch instabil werden und sich entmischen. Ein besonderer Vorteil der Verbindungen ergibt sich weiterhin dadurch, dass die Stabilität des Präparats ohne Zusatz von Emulgatoren erzielt werden kann. Dies ermöglicht eine Verbesserung der Verträglichkeit des erfindungsgemäßen Präparats.

In bevorzugten Ausführungsformen liegt die Viskosität des Präparats im Bereich von ≥ 400 mPa·s bis ≤ 7000 mPa·s, bevorzugt im Bereich von ≥ 500 mPa·s bis ≤ 1500 mPa·s, besonders bevorzugt im Bereich von ≥ 600 mPa·s bis ≤ 1400 mPa·s, ganz besonders bevorzugt im Bereich von ≥ 650 mPa·s bis ≤ 1300 mPa·s. Insbesondere ist von Vorteil, dass ein Präparat mit geringer Viskosität die Applizierbarkeit für den Patienten deutlich verbessert. Vorteilhafter Weise kann ein tropfbares Präparat zur Verfügung gestellt werden, das sich auf der Cornea des Auges verbessert verteilen kann, und das Auge weniger durch eine zusätzliche mechanische Unterstützung der Verteilung gereizt werden muss. Von großem Vorteil ist, dass das ophthalmische Präparat bei der Applikation ein nur geringes Fremdkörpergefühl im Auge erzeugt und für den Patienten eine angenehmere Verträglichkeit zur Verfügung stellen kann.

In bevorzugten Ausführungsformen weist das ophthalmische Präparat eine Osmolalität im Bereich im Bereich von ≥ 100 mosmol/kg bis ≤ 500 mosmol/kg, vorzugsweise im Bereich von ≥ 180 mosmol/kg bis ≤ 320 mosmol/kg, bevorzugt im Bereich von ≥ 200 mosmol/kg bis ≤ 280 mosmol/kg, besonders bevorzugt im Bereich von ≥ 220 mosmol/kg bis ≤ 260 mosmol/kg, auf.

In weiterhin bevorzugten Ausführungsformen weist das ophthalmische Präparat einen pH-Wert im Bereich von ≥ 6 bis ≤ 8 auf, bevorzugt weist das Präparat einen pH-Wert im Bereich von ≥ 6,5 bis ≤ 7,5, auf.

In besonders bevorzugten Ausführungsformen umfasst das ophthalmische Präparat Stoffe ausgewählt aus der Gruppe umfassend wenigstens ein Pflanzenöl ausgewählt aus der Gruppe umfassend Kiwisamenöl, Leinöl, Walnussöl, Rapsöl, Chiaöl, Perillaöl und/oder Hanföl, vorzugsweise Kiwisamenöl, Vitamin E, vorzugsweise gemischte Tocopherole, Polyacrylsäure, polymere Acrylsäurederivate, vorzugsweise Carbomer, wasserlösliche Phosphatsalze, vorzugsweise Na₂HPO₄ x 12 H₂O, Sorbitol, Natriumhydroxid und/oder Wasser.

In weiter besonders bevorzugten Ausführungsformen umfasst das ophthalmische Präparat, bezogen auf das Gesamtgewicht des Präparats, 0,2 Gew.-% polymere Acrylsäure, bevorzugt Carbomer, 1,0 Gew.-% Kiwisamenöl, 4,0 Gew.-% Sorbitol, 0,1 Gew.-% Na₂HPO₄ x 12 H₂O und/oder 0,063 Gew.-% Natriumhydroxid sowie ad 100 Gew.-% Wasser.

In bevorzugten Ausführungsformen umfasst das ophthalmische Präparat, bezogen auf das Gesamtgewicht des Präparats, 0,2 Gew.-% polymere Acrylsäure, bevorzugt Carbomer, 1,0 Gew.-% Pflanzenöl ausgewählt aus der Gruppe umfassend Kiwisamenöl, Leinöl, Walnussöl, Rapsöl, Chiaöl, Perillaöl und/oder Hanföl, vorzugsweise Kiwisamenöl, Sorbitol im Bereich von ≥ 4 Gew.-% bis ≤ 5 Gew.-%, Natriumhydroxid im Bereich von ≥ 0,06 Gew.-% bis ≤ 0,08 Gew.-%, gegebenenfalls 0,1 Gew.-% Na₂HPO₄ x 12 H₂O und/oder gegebenenfalls 0,01 Gew.-% Cetrimid sowie ad 100 Gew.-% Wasser.

In weiter bevorzugten Ausführungsformen umfasst das ophthalmische Präparat Stoffe ausgewählt aus der Gruppe umfassend wenigstens ein Pflanzenöl ausgewählt aus der Gruppe umfassend Kiwisamenöl, Leinöl, Walnussöl, Rapsöl, Chiaöl, Perillaöl und/oder Hanföl, vorzugsweise Kiwisamenöl, Polyacrylsäure, polymere Acrylsäurederivate, vorzugsweise Carbomer, wasserlösliche Phosphatsalze, vorzugsweise Na₂HPO₄ x 12 H₂O, Vitamin E, vorzugsweise gemischte Tocopherole, Sorbitol, Natriumhydroxid, Konservierungsmittel, vorzugsweise Cetrimid und/oder Wasser. In besonders bevorzugten Ausführungsformen umfasst das ophthalmische Präparat, bezogen auf das Gesamtgewicht des Präparats, 0,2 Gew.-% polymere Acrylsäure, bevorzugt Carbomer, 1,0 Gew.-% Kiwisamenöl, 4,0 Gew.-% Sorbitol, 0,1 Gew.-% Na₂HPO₄ x 12 H₂O, 0,01 Gew.-% Cetrimid und/oder 0,063 Gew.-% Natriumhydroxid sowie ad 100 Gew.-% Wasser.

In noch bevorzugten Ausführungsformen umfasst das ophthalmische Präparat Stoffe ausgewählt aus der Gruppe umfassend wenigstens ein Pflanzenöl ausgewählt aus der Gruppe umfassend Kiwisamenöl, Leinöl, Walnussöl, Rapsöl, Chiaöl, Perillaöl und/oder Hanföl, vorzugsweise Kiwisamenöl, Polyacrylsäure, polymere Acrylsäurederivate, vorzugsweise Carbomer, Vitamin E, vorzugsweise gemischte Tocopherole, Sorbitol, Natriumhydroxid, Konservierungsmittel, vorzugsweise Cetrimid, und/oder Wasser. In besonders bevorzugten Ausführungsformen umfasst das ophthalmische Präparat, bezogen auf das Gesamtgewicht des Präparats, 0,2 Gew.-% polymere Acrylsäure, bevorzugt Carbomer, 1,0 Gew.-% Kiwisamenöl, 0,01 Gew.-% Cetrimid, 4,85 Gew.-% Sorbitol und/oder 0,08 Gew.-% Natriumhydroxid sowie ad 100 Gew.-% Wasser.

In bevorzugten Ausführungsformen umfasst das Präparat, bezogen auf das Gesamtgewicht des Präparats, die nachstehenden Stoffe:
a. 0,2 Gew.-% polymere Acrylsäure;
b. 1,0 Gew.-% Kiwisamenöl;
c. 4,0 Gew.-% Sorbitol;
d. 0,1 Gew.-% Na₂HPO₄ x 12 H₂O;
e. 0,063 Gew.-% Natriumhydroxid;
f. ad 100 Gew.-% Wasser.

In besonders bevorzugten Ausführungsformen umfasst das Präparat, bezogen auf das Gesamtgewicht des Präparats, die nachstehenden Stoffe:
a. 0,2 Gew.-% polymere Acrylsäure;
b. 1,0 Gew.-% Kiwisamenöl;
c. 0,01 Gew.-% Cetrimid;
d. 4,0 Gew.-% Sorbitol;
e. 0,1 Gew.-% Na₂HPO₄ x 12 H₂O;
f. 0,063 Gew.-% Natriumhydroxid;
g. ad 100 Gew.-% Wasser. In auch bevorzugten Ausführungsformen umfasst das Präparat, bezogen auf das Gesamtgewicht des Präparats, die nachstehenden Stoffe:

a. 0,2 Gew.-% polymere Acrylsäure;
b. 1,0 Gew.-% Kiwisamenöl;
c. 0,01 Gew.-% Cetrimid;
d. 4,85 Gew.-% Sorbitol;
e. 0,08 Gew.-% Natriumhydroxid;
f. ad 100 Gew.-% Wasser.

Wenn nicht anders angegeben, sind die Gewichtsgehalte der jeweiligen im Präparat enthaltenen Stoffe so gewählt, dass das Gesamtgewicht der jeweiligen Stoffe 100 Gew.-%, bezogen auf das Gesamtgewicht des Präparats, nicht übersteigt.

Als weiteren Stoff kann das erfindungsgemäße Präparat Glycerol enthalten. Bei Glycerol handelt es sich im Sinne der vorliegenden Erfindung nicht um einen Emulgator, oder Glycerol ist in Mengen enthalten, dass der Stoff keine emulgierende Wirkung aufweist. Vorzugsweise kann das ophthalmische Präparat Glycerol im Bereich von ≥ 0,01 Gew.-% bis ≤ 1,0 Gew.-%, bezogen auf das Gesamtgewicht des Präparats, bevorzugt im Bereich von ≥ 0,1 Gew.-% bis ≤ 0,5 Gew.-%, besonders bevorzugt im Bereich von ≥ 0,2 Gew.-% bis ≤ 0,3 Gew.-%, aufweisen.

Unter dem Begriff "Stoff" sind im Sinne dieser Erfindung die Stoffe zu verstehen, die das Präparat aufweist, insbesondere ausgewählt aus der Gruppe umfassend polymere Acrylsäure, polymeres Acrylsäurederivat, Carbomer, Pflanzenöl ausgewählt aus der Gruppe umfassend Kiwisamenöl, Leinöl, Walnussöl, Rapsöl, Chiaöl, Perillaöl und/oder Hanföl, vorzugsweise Kiwisamenöl, Sorbitol, wasserlösliches Phosphatsalz, vorzugsweise Na₂HPO₄ x 12 H₂O, Natriumhydroxid, Konservierungsmittel, vorzugsweise Cetrimid, Vitamin E, vorzugsweise gemischte Tocopherole, eine Vitamin A-Komponente, vorzugsweise Vitamin A-palmitat, und/oder Wasser.

Das ophthalmische Präparat lässt sich vorteilhafter Weise unproblematisch aseptisch herstellen und ist hervorragend verträglich. Die Herstellung des erfindungsgemäßen sterilen Präparats, insbesondere Gelpräparats, erfolgt vorzugsweise in einem mehrstufigen Verfahren. Der wässrigen Phase des erfindungsgemäßen Präparats kann wenigstens ein wasserlösliches Phosphatsalz zusetzt werden.

Es versteht sich von selbst, dass das ophthalmische Präparat zur Verwendung vorzugsweise steril vorliegt, so dass sterile Stoffe unter sterilen Bedingungen verwendet werden, oder das Präparat nach der Einarbeitung der Stoffe sterilisiert wird.

Bei einem bevorzugten Verfahren zur Herstellung eines erfindungsgemäßen Präparats wird eine Suspension der polymeren Acrylsäure oder des polymeren Acrylsäurederivates, insbesondere eines Carbomers, hergestellt, bevorzugt unter aseptischen Bedingungen. Zu dieser Suspension wird anschließend eine wässrige sterilfiltrierte Lösung des Isotonisierungsmittels, vorzugsweise Sorbitol, enthaltend gegebenenfalls Natriummonohydrogenphosphatdodecahydrat, und/oder gegebenenfalls ein Konservierungsmittel gegeben, wobei unter Verwendung von bevorzugt sterilfiltiertem Stickstoff oder Druckluft als Druckgas gearbeitet werden kann. Nach sorgfältigem Durchmischen werden dann durch Zugabe einer geeigneten Base, vorzugsweise einer sterilen 2 %igen bis 3 %igen Natriumhydroxidlösung, die Carboxylgruppen der Polyacrylsäurekomponente neutralisiert, die Gelbildung der Polyacrylsäurekomponente eingeleitet. Es wird vorzugsweise bis zur Homogenität des Gels weitergerührt.

In das hergestellte sterile Hydrogel wird dann die hydrophobe flüssige Phase, umfassend wenigstens ein Pflanzenöl ausgewählt aus der Gruppe umfassend Kiwisamenöl, Leinöl, Walnussöl, Rapsöl, Chiaöl, Perillaöl und/oder Hanföl, vorzugsweise Kiwisamenöl, unter aseptischen Bedingungen eingearbeitet. Vorzugsweise wird das Pflanzenöl homogen in der kontinuierlichen wässrigen Phase, insbesondere dem sterilen Hydrogel, dispergiert.

Bevorzugt wird bis zur vollständigen Homogenisierung gerührt. Die Größe der so erzeugten Tröpfchen der hydrophobe flüssige Phase, oder Öltröpfchen, in der Dispersion liegt vorzugsweise bei maximal etwa 100 µm. Das sterile Präparat kann anschließend in üblicher Weise konfektioniert werden.

Bei dem Verfahren zur Herstellung eines erfindungsgemäßen Präparats kann ebenfalls ein Wirkstoff, wie Vitamin A-palmitat, dem so hergestellten sterilen Gel zugesetzt werden. Bevorzugt wird der Wirkstoff unter aseptischen Bedingungen zugesetzt und homogen eingemischt. Vorzugsweise wird die Vitamin A-Komponente und die vorzugsweise vorhandene sehr viel geringere Menge des Antioxidans in der hydrophoben Phase gelöst und dann steril filtriert. Die sterile hydrophobe wirkstoffhaltige Phase wird dann unter Rühren in das Gel eingearbeitet.

Von besonderem Vorteil ist, dass das erfindungsgemäße ophthalmische Präparat zur Herstellung eines kosmetischen und/oder pharmazeutischen Mittels zur Behandlung von Krankheiten oder Zuständen des Auges oder der das Auge umgebenden oder damit zusammenhängenden Organe oder Gewebe, insbesondere der Symptomatik des trockenen Auges verwendbar ist.

Durch die vorteilhaften Stoffe des Präparats kann dieses als Mittel zur Behandlung von Krankheiten oder Zuständen des Auges oder der das Auge umgebenden oder damit zusammenhängenden Organe oder Gewebe, insbesondere in Form eines Gelpräparats, eingesetzt werden. Insbesondere ist das Präparat zur Linderung der Beschwerden bei trockenem Auge und/oder für die symptomatische Behandlung des trockenen Auges geeignet.

Insbesondere ein konservierungsmittelfreies erfindungsgemäßes ophthalmisches Präparat ist in vorteilhafter Weise in der Langzeitanwendung bei trockenem Auge verwendbar. Durch die vorteilhaften Stoffe des ophthalmischen Präparats kann insbesondere ein konservierungsmittelfreies ophthalmisches Präparat unbedenklich zur andauernden Anwendung beispielsweise als Tränenersatzmittel Verwendung finden.

Es wurde überraschend gefunden, dass schon innerhalb weniger Minuten nach der Applikation des Präparats eine deutliche Besserung der Beschwerden wie Trockenheitsgefühl am Auge, aber auch Brennen, Jucken, Rötungen oder Schwellungen, bewirkt werden kann. Von besonderem Vorteil ist, dass diese Beschwerden nachhaltig durch das erfindungsgemäße Präparat gelindert werden können.

Ein weiterer Gegenstand der Erfindung betrifft entsprechend ein Mittel zur Behandlung von Krankheiten oder Zuständen des Auges oder der das Auge umgebenden oder damit zusammenhängenden Organe oder Gewebe, insbesondere der Symptomatik des trockenen Auges, umfassend Stoffe ausgewählt aus der Gruppe umfassend ein Pflanzenöl ausgewählt aus der Gruppe umfassend Kiwisamenöl, Leinöl, Walnussöl, Rapsöl, Chiaöl, Perillaöl und/oder Hanföl, Polyacrylsäure, polymere Acrylsäurederivate, vorzugsweise Carbomere, wasserlösliche Phosphatsalze, vorzugsweise Na₂HPO₄ x 12 H₂O, eine Vitamin A-Komponente, vorzugsweise Vitamin A-palmitat, Vitamin E, vorzugsweise gemischte Tocopherole, Sorbitol, Natriumhydroxid, Konservierungsmittel, vorzugsweise Cetrimid und/oder Wasser.

Für die Applikation wird das erfindungsgemäße Präparat vorzugsweise in geeignete Behältnisse abgefüllt, die vorzugsweise so gestaltet sind, dass der Inhalt auf das Auge aufgebracht werden kann, beispielsweise in Tropflaschen, insbesondere in unter der Bezeichnung Ophtiole® bekannte Behältnisse, vorzugsweise basierend auf Polyethylen, bevorzugt HDPE (Polyethylen hoher Dichte, high density Polyethylen) oder LDPE (Polyethylen niederer Dichte, low density Polyethylen).

In bevorzugten Ausführungsformen des ophthalmischen Präparats ist dieses in einer Eindosis-Einheit, vorzugsweise einem Einmaldosis-Behältnis beispielsweise bekannt unter der Bezeichnung Eindosis-Ophtiole®, vorzugsweise basierend auf LDPE, enthalten. In besonders bevorzugten Ausführungsformen des ophthalmischen Präparats liegt dieses in Form eines Gelpräparats in einem Einmaldosis-Behältnis frei von Konservierungsmitteln vor.

In weiterhin bevorzugten Ausführungsformen des sterilen tropfbaren mehrphasigen emulgatorfreien ophthalmischen Präparats ist dieses in einer Mehrdosen-Einheit, beispielsweise einem Mehrdosen-Behältnis enthalten. In auch besonders bevorzugten Ausführungsformen des ophthalmischen Präparats liegt dieses vorzugsweise in Form eines Gelpräparats in einem Mehrdosen-Behältnis frei von Konservierungsmitteln vor.

Eine Mehrdosen-Einheit enthält eine größere Menge des sterilen tropfbaren mehrphasigen emulgatorfreien ophthalmischen Präparats, beispielsweise mehrere Dosen. Somit ist vorteilhafter Weise möglich, dass beispielsweise eine Mehrfachdosis in einem Behältnis enthalten sein kann.

Die Viskosität wurde bestimmt nach dem Platte-Kegel-Verfahren mit einem Rheometer des Typs DV-III+ der Firma Brookfield, unter Verwendung einer CP51-Spindel, bei 5 U/min, bei 20 °C. Angaben der Viskosität beziehen sich, wenn nicht anders angegeben, auf die Viskosität des Präparats vor der Applikation am Auge.

Die folgenden Ausführungsbeispiele dienen der Erläuterung der Erfindung und stellen keinerlei Einschränkung dar.

### Beispiel 1

Augengel umfassend, bezogen auf 100 kg:
- 0,2 kg polymere Acrylsäure*¹;
- 1 kg Kiwisamenöl;
- 0,01 kg Cetrimid;
- 4,0 kg Sorbitol;
- 0,1 kg Na₂HPO₄ x 12 H₂O
- 0,063 kg Natriumhydroxid;
- ad 100 kg Wasser.
*¹ Beispielsweise erhältlich unter dem Handelsnamen Carbopol® 980 NF bei der Firma Noveon Inc..

### Beispiel 2

Augengel umfassend, bezogen auf 100 kg:
- 0,2 kg polymere Acrylsäure*¹;
- 1 kg Kiwisamenöl;
- 4,0 kg Sorbitol;
- 0,1 kg Na₂HPO₄ x 12 H₂O;
- 0,063 kg Natriumhydroxid;
- ad 100 kg Wasser.
*¹ Beispielsweise erhältlich unter dem Handelsnamen Carbopol® 980 NF bei der Firma Noveon Inc..

### Beispiel 3

Augengel umfassend, bezogen auf 100 kg:
- 0,2 kg polymere Acrylsäure*¹;
- 1 kg Kiwisamenöl;
- 0,01 kg Cetrimid;
- 4,851 kg Sorbitol;
- 0,08 kg Natriumhydroxid;
- ad 100 kg Wasser.
*¹ Beispielsweise erhältlich unter dem Handelsnamen Carbopol® 980 NF bei der Firma Noveon Inc..

## Patentansprüche

1. Steriles tropfbares mehrphasiges emulgatorfreies ophthalmisches Präparat, insbesondere Gelpräparat, enthaltend wenigstens eine flüssige wässrige Phase und wenigstens eine flüssige hydrophobe Phase,
**dadurch gekennzeichnet, dass** das Präparat wenigstens ein Pflanzenöl ausgewählt aus der Gruppe umfassend Kiwisamenöl, Leinöl, Walnussöl, Rapsöl, Chiaöl, Perillaöl und/oder Hanföl umfasst.

2. Präparat nach Anspruch 1, **dadurch gekennzeichnet, dass**
das Präparat wenigstens ein Pflanzenöl ausgewählt aus der Gruppe umfassend Kiwisamenöl, Leinöl, Walnussöl, Rapsöl, Chiaöl, Perillaöl und/oder Hanföl im Bereich von ≥ 0,01 Gew.-% bis ≤ 10 Gew.-%, bezogen auf das Gesamtgewicht des Präparats, bevorzugt im Bereich von ≥ 0,1 Gew.-% bis ≤ 5 Gew.-%, vorzugsweise im Bereich von ≥ 0,5 Gew.-% bis ≤ 2 Gew.-%, besonders bevorzugt etwa 1 Gew.-%, enthält.

3. Präparat nach Anspruch 1 oder 2, **dadurch gekennzeichnet, dass**
das Präparat wenigstens eine polymere gelbildende Komponente, vorzugsweise eine Polyacrylsäure und/oder ein polymeres Acrylsäurederivat, bevorzugt ein Carbomer enthält.

4. Präparat nach einem der vorherigen Ansprüche, **dadurch gekennzeichnet, dass** das Präparat ein Phosphatsalz, bevorzugt ein wasserlösliches Phosphatsalz enthält, vorzugsweise ausgewählt aus der Gruppe umfassend Alkaliphosphatsalze, Erdalkaliphosphatsalze, Ammoniumphosphatsalze, deren Mono- oder Dihydrogenphosphate und/oder Mischungen davon, bevorzugt ausgewählt aus der Gruppe umfassend Natriumdihydrogenphosphat, Natriummonohydrogenphosphat, Kaliumdihydrogenphosphat, Kaliummonohydrogenphosphat und/oder Mischungen davon, besonders bevorzugt Na₂HPO₄ x 12 H₂O.

5. Präparat nach einem der vorherigen Ansprüche, **dadurch gekennzeichnet, dass** das Präparat wenigstens einen ophthalmischen Wirkstoff enthält, vorzugsweise ausgewählt aus der Gruppe umfassend Vitamin A und/oder Vitamin E, bevorzugt Vitamin A-palmitat und/oder Tocopherole.

6. Präparat nach einem der vorherigen Ansprüche, **dadurch gekennzeichnet, dass** das Präparat Sorbitol aufweist.

7. Präparat nach einem der vorherigen Ansprüche, **dadurch gekennzeichnet, dass** das Präparat Natriumhydroxid aufweist.

8. Präparat nach einem der vorherigen Ansprüche, **dadurch gekennzeichnet, dass** das Präparat Konservierungsmittel aufweist, vorzugsweise ausgewählt aus der Gruppe umfassend Cetrimid, Benzododeciniumchlorid, Benzalkoniumchlorid und/oder Thiomersal.

9. Präparat nach einem der vorherigen Ansprüche, **dadurch gekennzeichnet, dass** die Viskosität des Präparats im Bereich von ≥ 300 mPa·s bis ≤ 8000 mPa·s, vorzugsweise im Bereich von ≥ 400 mPa·s bis ≤ 7000 mPa·s, bevorzugt im Bereich von ≥ 500 mPa·s bis ≤ 1500 mPa·s, besonders bevorzugt im Bereich von ≥ 600 mPa·s bis ≤ 1400 mPa·s, ganz besonders bevorzugt im Bereich von ≥ 650 mPa·s bis ≤ 1300 mPa·s, liegt.

10. Präparat nach einem der vorherigen Ansprüche, **dadurch gekennzeichnet, dass** das Präparat eine Osmolalität im Bereich im Bereich von ≥ 100 mosmol/kg bis ≤ 500 mosmol/kg, vorzugsweise im Bereich von ≥ 180 mosmol/kg bis ≤ 320 mosmol/kg, bevorzugt im Bereich von ≥ 200 mosmol/kg bis ≤ 280 mosmol/kg, besonders bevorzugt im Bereich von ≥ 220 mosmol/kg bis ≤ 260 mosmol/kg, aufweist.

11. Präparat nach einem der vorherigen Ansprüche, **dadurch gekennzeichnet, dass** das Präparat einen pH-Wert im Bereich von ≥ 6 bis ≤ 8, bevorzugt im Bereich von ≥ 6,5 bis ≤ 7,5, aufweist.

12. Präparat nach einem der vorherigen Ansprüche, **dadurch gekennzeichnet, dass** das Präparat Stoffe ausgewählt aus der Gruppe umfassend wenigstens ein Pflanzenöl ausgewählt aus der Gruppe umfassend Kiwisamenöl, Leinöl, Walnussöl, Rapsöl, Chiaöl, Perillaöl und/oder Hanföl, Polyacrylsäure, polymere Acrylsäurederivate, vorzugsweise Carbomere, wasserlösliche Phosphatsalze, vorzugsweise Na₂HPO₄ x 12 H₂O, eine Vitamin A-Komponente, vorzugsweise Vitamin A-palmitat, Vitamin E, vorzugsweise gemischte Tocopherole, Sorbitol, Natriumhydroxid, Konservierungsmittel, vorzugsweise Cetrimid oder Benzododeciniumchlorid und/oder Wasser umfasst.

13. Präparat nach einem der vorherigen Ansprüche, **dadurch gekennzeichnet, dass** das Präparat, bezogen auf das Gesamtgewicht des Präparats, die nachstehenden Stoffe umfasst:
a. 0,2 Gew.-% polymere Acrylsäure;
b. 1,0 Gew.-% Kiwisamenöl;
c. 4,0 Gew.-% Sorbitol;
d. 0,1 Gew.-% Na₂HPO₄ x 12 H₂O;
e. 0,063 Gew.-% Natriumhydroxid;
f. ad 100 Gew.-% Wasser.

14. Präparat nach einem der vorherigen Ansprüche, **dadurch gekennzeichnet, dass** das Präparat, bezogen auf das Gesamtgewicht des Präparats, die nachstehenden Stoffe umfasst:
a. 0,2 Gew.-% polymere Acrylsäure;
b. 1,0 Gew.-% Kiwisamenöl;
c. 0,01 Gew.-% Cetrimid;
d. 4,0 Gew.-% Sorbitol;
e. 0,1 Gew.-% Na₂HPO₄ x 12 H₂O;
f. 0,063 Gew.-% Natriumhydroxid;
g. ad 100 Gew.-% Wasser.

15. Präparat nach einem der vorherigen Ansprüche, **dadurch gekennzeichnet, dass** das Präparat, bezogen auf das Gesamtgewicht des Präparats, die nachstehenden Stoffe umfasst:
a. 0,2 Gew.-% polymere Acrylsäure;
b. 1,0 Gew.-% Kiwisamenöl;
c. 0,01 Gew.-% Cetrimid;
d. 4,85 Gew.-% Sorbitol;
e. 0,08 Gew.-% Natriumhydroxid;
f. ad 100 Gew.-% Wasser.

16. Verwendung eines Präparats nach einem der vorherigen Ansprüche zur Herstellung eines kosmetischen und/oder pharmazeutischen Mittels zur Behandlung von Krankheiten oder Zuständen des Auges oder der das Auge umgebenden oder damit zusammenhängenden Organe oder Gewebe, insbesondere der Symptomatik des trockenen Auges.

17. Mittel zur Behandlung von Krankheiten oder Zuständen des Auges oder der das Auge umgebenden oder damit zusammenhängenden Organe oder Gewebe, insbesondere der Symptomatik des trockenen Auges, umfassend Stoffe gemäß einem der vorherigen Ansprüche.

18. Einmaldosis-Behältnis enthaltend ein Präparat nach einem der vorherigen Ansprüche.

19. Einmaldosis-Behältnis nach Anspruch 18, **dadurch gekennzeichnet, dass** das Präparat frei von Konservierungsmitteln ist.

20. Mehrdosen-Behältnis enthaltend ein Präparat nach einem der vorherigen Ansprüche.

21. Mehrdosen-Behältnis nach Anspruch 20, **dadurch gekennzeichnet, dass** das Präparat frei von Konservierungsmitteln ist.
